# FASCICULE DE BREVET EUROPEEN

(11) **EP 1 555 013 B1**
(45) Date de publication et mention de la délivrance du brevet: **27.06.2007**
(21) Numéro de dépôt: 04292963.8
(22) Date de dépôt: 13.12.2004
(51) Int. Cl.: A61K 8/22, A61K 8/68, A61Q 5/08

(54) **Pâté anhydre de décoloration comprenant au moins un composé de type céramide**
Wasserfreie Paste zur Haarentfärbung mindestens eine Verbindung des Types Ceramid enthaltend
Anhydrous bleaching paste containing at least one ceramid-type compound

(30) Priorité: 29.12.2003 FR 0351216
(43) Date de publication de la demande: 20.07.2005
(73) Titulaire: L'ORÉAL, 75008 Paris (FR)
(72) Inventeur: Kravtchenko, Sylvain, 92600 Asnières (FR); Legrand, Frédéric, 92400 Courbevoie (FR)
(74) Mandataire: Fevrier, Murielle Françoise E.

(56) Documents cités:
- EP-A- 0 879 593
- EP-A- 1 023 891
- EP-A- 1 228 751
- WO-A-97/15273
- DE-A1- 3 814 356
- FR-A- 2 802 092

## Description

La présente invention a pour objet une pâte anhydre pour la décoloration des fibres kératiniques humaines, comprenant au moins un composé de type céramide. Elle concerne de plus un procédé mettant en oeuvre ladite composition ainsi qu'un dispositif approprié à plusieurs compartiments .

La décoloration des fibres kératiniques humaines, et plus particulièrement des cheveux, se fait par oxydation du pigment "mélanine" aboutissant à la solubilisation et l'élimination partielle ou totale de ce pigment.

Pour décolorer les cheveux, on utilise une composition décolorante, sous forme de poudre ou plus avantageusement sous forme de pâte anhydre, contenant au moins un réactif peroxygéné, que l'on associe immédiatement avant l'emploi à une composition aqueuse de peroxyde d'hydrogène.

Les sels peroxygénés et le peroxyde d'hydrogène étant relativement stables en milieu acide, il est souvent nécessaire de les activer à pH basique pour obtenir une formation adéquate d'oxygène. Il est donc usuel d'ajouter à la composition décolorante, des composés alcalins comme l'urée, les silicates et les phosphates alcalins ou alcalino-terreux ou encore des agents précurseurs d'ammoniac.

Le problème rencontré avec l'usage de ce type de composition réside dans le fait qu'à la longue, on observe une dégradation de la fibre kératinique. Cela se traduit notamment par une diminution des propriétés cosmétiques de la fibre, perte de douceur par exemple, et par une diminution des propriétés mécaniques de cette dernière. En effet, la fibre est fragilisée et l'on observe un taux de casse de la fibre lors du démêlage, par exemple.

On a proposé de mettre en oeuvre des compositions de décoloration sous forme de poudre, comprenant des persels et un composé de type céramide. Si de telles compositions constituent une amélioration par rapport à des compostions pulvérulentes dépourvues de céramide, les résultats obtenus ne sont toutefois pas entièrement satisfaisants.

La présente invention a donc pour objet de proposer des compositions permettant d'éclaircir de manière intense les cheveux tout en limitant leur dégradation tout en offrant une sécurité accrue et des performances améliorées en terme d'éclaircissement et de confort.

Ces buts et d'autres sont atteints par la présente invention qui a pour premier objet une pâte anhydre pour la décoloration des fibres kératiniques humaines, et plus particulièrement des cheveux, comprenant au moins un liquide inerte organique, au moins un sel peroxygéné et au moins un agent alcalin, caractérisée en ce qu'elle comprend au moins un composé de type céramide.

Un autre objet de l'invention est constitué par un procédé de décoloration des fibres kératiniques humaines, et en particulier les cheveux, comprenant les étapes consistant :
- à mélanger, immédiatement avant emploi, une pâte anhydre selon l'invention, avec une composition aqueuse de peroxyde d'hydrogène,
- à appliquer le mélange obtenu sur la zone des fibres à décolorer,
- à laisser poser pendant un temps suffisant pour obtenir la décoloration recherchée,
- à éliminer le mélange décolorant par rinçage à l'eau suivi d'un lavage avec un shampooing, puis éventuellement d'un séchage.

L'invention concerne de plus un dispositif à plusieurs compartiments pour la décoloration des fibres kératiniques humaines et plus particulièrement des cheveux, caractérisé en ce qu'il comporte au moins deux compartiments dont l'un comprend une pâte anhydre selon l'invention, et l'autre une composition aqueuse de peroxyde d'hydrogène.

Mais d'autres caractéristiques et avantages de la présente invention apparaîtront plus clairement à la lecture de la description et de l'exemple qui vont suivre.

Ainsi que cela a été indiqué précédemment, la pâte anhydre, objet de l'invention, comprend au moins un composé de type céramide.

Il est indiqué que par le terme "anhydre", on désigne une pâte dont la teneur en eau est inférieure à 1%, et de préférence inférieure à 0,5% en poids par rapport au poids total de la pâte.

Plus particulièrement, on appelle composé de type céramide, un composé choisi parmi les céramides et/ou les glycocéramides et/ou les pseudocéramides.

Selon un mode de réalisation avantageux de la présente invention, le composé de type céramide est choisi parmi les composés correspondant à la formule (A) suivante : 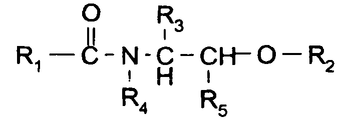 dans laquelle :
**R₁** désigne:
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifiés par un acide gras saturé ou insaturé, linéaire ou ramifié, éventuellement mono ou polyhydroxylé, en C₁-C₃₅,
   - soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
   - soit un radical R₈-O-CO-(CH₂)ₚ, R₈ désignant un radical hydrocarboné en C₁-C₂₀, p étant un entier variant de 1 à 12 ;
**R₂** est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
**R₃** désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthyl ammonium, R₃ pouvant également être substitué par un ou plusieurs radicaux alkyle en C₁-C₁₄ ;
**R₄** désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p étant un entier variant de 1 à 12,
**R₅** désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (A) ci-dessus, on peut citer tout particulièrement les céramides et/ou glycocéramides décrits par DOWNING dans Journal of Lipid Research, Vol. 35, page 2060, 1994 ou ceux décrits dans la demande de brevet français FR-2 673 179, et dont les enseignements sont ici inclus à titre de référence.

Parmi les composés de type céramide préférés, on peut citer ceux pour lesquels, dans la formule (A), R₁ désigne un radical alkyle saturé ou insaturé dérivé d'acides gras en C₁₄-C₂₂ éventuellement hydroxylé; R₂ désigne un atome d'hydrogène ; et R₃ désigne un radical linéaire saturé en C₁₁-C₁₇ éventuellement hydroxylé et de préférence en C₁₃-C₁₅.

De tels composés sont par exemple choisis parmi, seuls ou en mélange :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
- le N-palmitamidohexadécanediol,

On peut aussi utiliser des mélanges spécifiques tels que par exemple les mélanges de céramide(s) 2 et de céramide(s) 5 selon la classification de DOWNING.

On peut également utiliser les composés de formule (A) pour lesquels R₁ désigne un radical alkyle saturé ou insaturé dérivé d'acides gras ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

Des composés de type céramide sont par exemple décrits dans les demandes de brevet DE 4424530, DE 4424533, DE 4402929, DE 4420736, WO 95/23807, WO 94/07844, EP 646572, WO 95/16665, FR 2673179, EP 227994 et WO 94/07844, WO 94/24097, WO 94/10131 auxquels on pourra se référer.

A titre d'exemple, on peut citer le produit constitué d'un mélange de glycocéramides, vendu sous la dénomination commerciale Glycocer® par la société Waitaki International Biosciences.

On peut également utiliser les composés décrits dans les demandes de brevet EP 227994, EP 647617, EP 736522 et WO 94/07844.

De tels composés sont par exemple le Ouestamide H®, encore appelé bis-(N-hydroxyéthyl N-cétyl) malonamide et vendu par la société Quest et le N-(2-hydroxyéthyl)-N-(3-cétyloxy-2-hydroxypropyl)amide d'acide cétylique .

On peut également utiliser le N-docosanoyl N-méthyl-D-glucamine tel que décrit dans la demande internationale WO 94/24097.

Selon un mode de réalisation avantageux de l'invention, la teneur en composé de type céramide représente de 0,001 à 5 % en poids, plus particulièrement de 0,01 à 3 % en poids et de préférence de 0,05 à 1 %, en poids par rapport au poids de la pâte anhydre.

Outre le composé de type céramide, la pâte anhydre de décoloration comprend au moins un liquide organique inerte.

Plus particulièrement, le liquide inerte organique est choisi parmi par les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales.

Les composés de formule C₁₀ₙ H_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe.

Ce sont des produits d'hydrogénation des poly-1-décènes.

Parmi ces composés, on choisit plus particulièrement selon l'invention ceux pour lesquels dans la formule, n varie de 3 à 7.

On peut citer, à titre d'exemple, et de préférence, le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les esters d'alcools gras ou d'acides gras, on désigne notamment :
- les esters de monoalcools inférieurs saturés linéaires ou ramifiés en C₃-C₆, avec des acides gras monofonctionnels en C₁₂-C₂₄ (ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates, etc...). Parmi lesdits esters, on préfère plus particulièrement utiliser le palmitate d'isopropyle, le myristate d'isopropyle et le stéarate d'octyl dodécyle.
- les esters de monoalcools linéaires ou ramifiés en C₃-C₈, avec des acides gras bifonctionnels en C₈-C₂₄ (ces derniers pouvant être linéaires ou ramifiés, saturés ou insaturés), comme par exemple le di-ester isopropylique de l'acide sébacique (sébaçate de di-isopropyle),
- les esters de monoalcools linéaires ou ramifiés en C₃-C₈, avec des acides gras bifonctionnels en C₂-C₈ (ces derniers pouvant être linéaires ou ramifiés, saturés ou insaturés), comme par exemple l'adipate de di-octyle et le maléate de di-caprylyle,
- l'ester d'un acide trifonctionnnel comme le citrate de tri-éthyle.

Le liquide inerte organique peut aussi être choisi parmi les esters et di-esters de sucres d'acides gras en C₁₂-C₂₄.

Par "sucre", on entend des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Lesdits esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates, etc...

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer, à titre d'exemple, et de préférence, le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose;
- les produits vendus sous la dénomination Ryoto Sugar Esters par exemple référencés B370 et correspondant au béhénate de saccharose formé de 20% de monoester et 80% de di-triester-polyester;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme liquide organique inerte.

Plus particulièrement, les huiles de silicone convenables sont des fluides de silicones liquides et non volatiles de viscosité inférieure ou égale à 10 000 mPa.s à 25°C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment par exemple les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme liquide inerte organique, comme par exemple l'huile de paraffine.

Les huiles végétales peuvent aussi convenir, et notamment l'huile d'avocat, l'huile d'olive ou la cire liquide de jojoba.

De préférence, le liquide inerte organique est choisi dans le groupe formé par les polydécènes de formule C₁₀ₙH_{[(20n)+2]} dans laquelle n varie de 3 à 9 et de préférence de 3 à 7, et les esters d'alcools gras ou d'acides gras.

Par ailleurs, conformément à un mode de réalisation de l'invention, la teneur en liquide inerte organique varie de 5 à 60 % en poids, de préférence de 10 à 50 % en poids par rapport au poids de la pâte anhydre.

Comme indiqué auparavant, la pâte anhydre selon l'invention comprend au moins un sel peroxygéné.

Avantageusement, ce dernier est choisi parmi les persulfates, perborates et percarbonates, d'ammonium ou de métaux alcalins, comme le potassium, le sodium ; le peroxyde de magnésium ; seul ou en mélange.

De préférence, la pâte anhydre comprend au moins un persulfate comme sel peroxygéné, et de manière encore plus préférée, au moinsun persulfate de sodium et de potassium.

Conformément à une variante de l'invention, la teneur en sel peroxygéné varie de 10 à 70 % en poids, de préférence de 20 à 60 % en poids par rapport au poids de la pâte anhydre.

La pâte anhydre selon l'invention comprend de plus, au moins un agent alcalin. De préférence, cet agent est choisi parmi l'urée, les silicates, phosphates et bicarbonates de métaux alcalins ou alcalino-terreux, et en particulier les métasilicates de métaux alcalins, ou des agents précurseurs d'ammoniac tels que les sels d'ammonium comme les chlorures, sulfates, phosphates ou nitrates.

Habituellement, la teneur en agent alcalin varie de 0,01 à 40 % en poids, de préférence de 0,1 à 30 % en poids par rapport au poids de la pâte anhydre.

La pâte anhydre selon l'invention peut de même comprendre d'autres additifs usuels dans le domaine.

La pâte décolorante peut ainsi comprendre des polymères épaississants hydrosolubles, des charges telles que des argiles ou de la silice amorphe, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs anioniques, non ioniques cationiques ou amphotères, des vitamines.

A titre illustratif, la teneur en additif(s) représentent 0,01 à 40 % en poids, de préférence de 0,1 à 30% en poids par rapport au poids total de la composition.

La pâte anhydre décolorante qui vient d'être décrite peut être obtenue en dispersant, sous action mécanique, l'ensemble des composés pulvérulents dans le liquide inerte organique, dans lequel on a préalablement dispersé ou mélangé les autres composés liquides de la composition.

On peut également préparer la pâte par extrusion, en introduisant les phases liquides et solides de la composition dans l'extrudeur, puis en les mélangeant à une température inférieure à 25°C à l'aide d'un système bi-vis co-rotatives composé d'éléments de transport et de malaxage.

La pâte décolorante anhydre qui vient d'être décrite est avantageusement utilisée pour la préparation d'une composition prête à l'emploi qui résulte du mélange extemporané de ladite pâte avec une composition aqueuse de peroxyde d'hydrogène.

La pâte anhydre est ainsi mélangée avec environ 0,5 à 10 équivalents en poids d'une composition aqueuse de peroxyde d'hydrogène qui peut être une solution, une émulsion, directe ou inverse, ou un gel, ayant une concentration pondérale allant de 2 à 12% en peroxyde d'hydrogène.

Ce mélange doit se faire immédiatement avant l'application du produit sur les cheveux.

La composition aqueuse de peroxyde d'hydrogène présente de préférence un pH inférieur à 7 ; le pH acide garantissant la stabilité du peroxyde d'hydrogène dans cette composition.

Il peut être obtenu à l'aide d'agents acidifiants tels que par exemple l'acide chlorhydrique, l'acide acétique, l'acide éthydronique, l'acide phosphorique, l'acide lactique ou l'acide borique, et il peut être ajusté classiquement par ajout soit d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

La composition aqueuse de peroxyde d'hydrogène peut encore contenir des agents conservateurs, des colorants, des parfums, des agents antimousse, des agents stabilisants du peroxyde d'hydrogène tels que notamment le pyrophosphate de sodium, le stannate de sodium et le salicylate de sodium ainsi que des agents séquestrants tels que par exemple l'acide éthylènediamine tétraacétique (EDTA) ou le Pentasodium Pentetate (dénomination CTFA).

Bien entendu, l'homme de l'art veillera à choisir le ou les éventuels composés complémentaires mentionnés ci-avant, de manière telle que les propriétés avantageuses attachées intrinsèquement à la pâte anhydre de décoloration ou à la composition de décoloration prête à l'emploi selon l'invention ne soient pas, ou substantiellement pas, altérées par la ou les adjonctions envisagées.

Le pH de la composition de décoloration prête à l'emploi est généralement compris entre les valeurs 4 et 12. Il est de préférence compris entre 7 et 11,5 et encore plus préférentiellement entre 8 et 11.

Le procédé de décoloration selon l'invention consiste à mélanger immédiatement avant emploi une pâte anhydre telle que décrite, avec une composition aqueuse de peroxyde d'hydrogène. Puis on applique la composition de décoloration prête à l'emploi ainsi obtenue sur la zone des fibres kératiniques humaines, sèches ou humides, à décolorer, et à la laisser agir pendant un temps de pose suffisant pour obtenir la décoloration recherchée.

En général, cette durée varie de 1 à 60 minutes environ, et de préférence de 1 à 30 minutes environ.

Habituellement, la température à laquelle on applique la composition est de l'ordre de 15 à 80°C et de préférence de 15 à 40°C.

Une fois la décoloration recherchée obtenue, on élimine le mélange décolorant par rinçage à l'eau des fibres, suivi de préférence d'au moins un lavage avec un shampooing, puis éventuellement d'un séchage.

Selon un autre aspect, l'invention a aussi pour objet un dispositif à plusieurs compartiments pour la décoloration des fibres kératiniques humaines, et plus particulièrement des cheveux, comprenant au moins deux compartiments dont l'un contient une pâte anhydre selon l'invention, et l'autre une composition aqueuse de peroxyde d'hydrogène.

Un exemple concret mais non limitatif va maintenant être présenté.

### Exemple

On prépare la composition suivante :

| | **Composition (g)** |
|---|---|
| Copolymère steareth-100/PEG-136 /HMDI | 2,0 |
| Myristate d'Isopropyle | 21,6 |
| Gomme xanthane | 1,4 |
| Ultramarines | 0,5 |
| oxide de magnésium | 2,0 |
| Silicate de sodium | 15,0 |
| Dioxide de titane | 1,0 |
| N-oléyl dihydrosphingosine | 0,01 |
| Stéarate de magnésium | 2,0 |
| EDTA | 0,2 |
| Sodium lauryl sulfate | 4,0 |
| Silice | 1,0 |
| Persulfate de potassium | 39,08 |
| Persulfate de sodium | 6,0 |
| Cire d'abeille | 1,2 |
| Huile minérale | 1,0 |
| Sel de sodium d'amidon carboxyméthylé | 2,0 |

La composition est mélangée avec de l'oxydant 30 volumes.

Il s'agit d'un oxydant de composition suivante :

| | |
|---|---|
| Stannate de sodium | 0,04 |
| Pentétate de sodium | 0,06 |
| Alcool cétéarylique | 8,00 |
| ceteareth-33 | 2,00 |
| Acide phosphorique | qsp pH 3 |
| Peroxyde d'hydrogène | 9,00 |
| Pyrophosphate de sodium | 0,03 |
| Copolymère acrylates/beheneth-25 méthacrylate (Aculyn 28® commercialise par Rhom & Haas) | 0,40 |
| Eau | 80,47 |

Le mélange est facile à obtenir et à appliquer. Il est réparti sur des mèches de cheveux naturels châtains de 2,7g.

Le rapport de bain est de 10 et le temps de pose de 35 minutes, après quoi les mèches sont rincées et shampouinées.

Il apparaît que les cheveux ont un niveau de décoloration excellent.

## Revendications

1. Pâte anhydre pour la décoloration des fibres kératiniques humaines, et plus particulièrement des cheveux, comprenant au moins un liquide inerte organique, au moins un sel peroxygéné et au moins un agent alcalin, **caractérisée en ce qu'**elle comprend au moins un composé de type céramide.

2. Pâte anhydre selon la revendication précédente, **caractérisée en ce que** le composé de type céramide est choisi parmi les céramides et/ou les glycocéramides et/ou les pseudocéramides.

3. Pâte anhydre selon l'une quelconque des revendications précédentes, **caractérisée en ce que** le composé de type céramide est choisi parmi les composés correspondant à la formule (A) suivante : 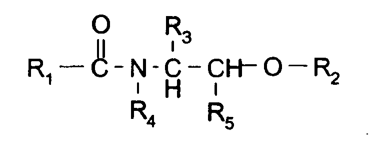 dans laquelle :
R₁ désigne:
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifiés par un acide gras saturé ou insaturé, linéaire ou ramifié, éventuellement mono ou polyhydroxylé, en C₁-C₃₅,
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
- soit un radical R₈-O-CO-(CH₂)ₚ, R₈ désignant un radical hydrocarboné en C₁-C₂₀, p étant un entier variant de 1 à 12 ;
R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthyl ammonium, R₃ pouvant également être substitué par un ou plusieurs radicaux alkyle en C₁-C₁₄;
R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p étant un entier variant de 1 à 12,
R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthyl ammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

4. Pâte anhydre selon l'une quelconque des revendications précédentes, **caractérisée en ce que** la teneur en composé de type céramide représente de 0,001 à 5 % en poids, plus particulièrement de 0,01 à 3 % en poids et de préférence de 0,05 à 1 %, en poids par rapport au poids de la pâte anhydre.

5. Pâte anhydre selon l'une quelconque des revendications précédentes, **caractérisée en ce que** le liquide inerte organique est choisi parmi par les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales.

6. Pâte selon la revendication précédente, **caractérisée en ce que** le liquide inerte organique est choisi dans le groupe formé par les polydécènes de formule C₁₀ₙH_{[(20n)+2]} dans laquelle n varie de 3 à 9 et de préférence de 3 à 7, et les esters d'alcools gras ou d'acides gras.

7. Pâte anhydre selon l'une quelconque des revendications précédentes, **caractérisée en ce que** la teneur en liquide inerte organique varie de 5 à 60 % en poids, de préférence de 10 à 50 % en poids par rapport au poids de la pâte anhydre.

8. Pâte anhydre selon l'une quelconque des revendications précédentes, **caractérisée en ce que** le sel peroxygéné est choisi parmi les persulfates, perborates et percarbonates, d'ammonium ou de métaux alcalins, comme le potassium, le sodium ; le peroxyde de magnésium ; seul ou en mélange.

9. Pâte anhydre selon la revendication précédente, **caractérisée en ce que** la teneur en sel peroxygéné varie de 10 à 70 % en poids, de préférence de 20 à 60 % en poids par rapport au poids de la pâte anhydre.

10. Pâte anhydre selon l'une quelconque des revendications précédentes, **caractérisée en ce que** l'agent alcalin est choisi parmi l'urée, les silicates, les phosphates et bicarbonates de métaux alcalins ou alcalino-terreux, des agents précurseurs d'ammoniac.

11. Pâte anhydre selon la revendication précédente, **caractérisée en ce que** la teneur en agent alcalin varie de 0,01 à 40 % en poids, de préférence de 0,1 à 30 % en poids par rapport au poids de la pâte anhydre.

12. Procédé de décoloration des fibres kératiniques humaines, et en particulier des cheveux, comprenant les étapes consistant :
- à mélanger, immédiatement avant emploi, une pâte anhydre définie selon l'une quelconque des revendications 1 à 11, avec une composition aqueuse de peroxyde d'hydrogène,
- à appliquer le mélange obtenu sur la zone des fibres à décolorer,
- à laisser poser pendant un temps suffisant pour obtenir la décoloration recherchée,
- à éliminer le mélange décolorant par rinçage à l'eau suivi d'un lavage avec un shampooing, puis éventuellement d'un séchage.

13. Dispositif à plusieurs compartiments pour la décoloration des fibres kératiniques humaines et plus particulièrement des cheveux, **caractérisé en ce qu'**il comporte au moins deux compartiments dont l'un comprend une pâte anhydre selon l'une quelconque des revendications 1 à 11, et l'autre une composition aqueuse de peroxyde d'hydrogène.

## Claims

1. Anhydrous paste for bleaching human keratinous fibres, and more particularly the hair, comprising at least one organic inert liquid, at least one peroxygenated salt and at least one alkaline agent, **characterized in that** it comprises at least one ceramide-type compound.

2. Anhydrous paste according to the preceding claim, **characterized in that** the ceramide-type compound is chosen from ceramides and/or glycoceramides and/or pseudoceramides.

3. Anhydrous paste according to either of the preceding claims, **characterized in that** the ceramide-type compound is chosen from the compounds corresponding to the following formula (A): 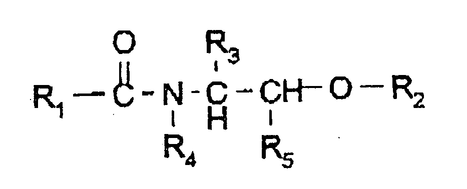 in which:
R₁ denotes:
- either a saturated or unsaturated, linear or branched C₁-C₅₀, preferably C₅-C₅₀, hydrocarbon radical, it being possible for this radical to be substituted with one or more hydroxyl groups optionally esterified with an acid R₇COOH, R₇ being an optionally mono- or polyhydroxylated, linear or branched, saturated or unsaturated C₁-C₃₅ hydrocarbon radical, it being possible for the hydroxyl(s) of the radical R₇ to be esterified with an optionally mono- or polyhydroxylated, linear or branched, saturated or unsaturated C₁-C₃₅ fatty acid,
- or a radical R"- (NR-CO) -R', R denotes a hydrogen atom or a mono- or polyhydroxylated, preferably monohydroxylated, C₁-C₂₀ hydrocarbon radical, R' and R" are hydrocarbon radicals of which the sum of the carbon atoms is between 9 and 30, R' being a divalent radical,
- or a radical R₈-O-CO-(CH₂)ₚ, R₈ denoting a C₁-C₂₀ hydrocarbon radical, p being an integer varying from 1 to 12;
R₂ is chosen from a hydrogen atom, a saccharide-type radical, in particular a (glycosyl)ₙ, (galactosyl)ₘ and sulphogalactosyl radical, a sulphate or phosphate residue, a phosphorylethylamine radical and a phosphorylethylammonium radical, in which n is an integer varying from 1 to 4 and m is an integer varying from 1 to 8;
R₃ denotes a hydrogen atom or a hydroxylated or nonhydroxylated, saturated or unsaturated, C₁-C₃₃ hydrocarbon radical, it being possible for the hydroxyl(s) to be esterified with an inorganic acid or an acid R₇COOH, R₇ having the same meanings as above, and it being possible for the hydroxyl(s) to be etherified with a (glycosyl)ₙ, (galactosyl)ₘ, sulphogalactosyl, phosphorylethylamine or phosphorylethylammonium radical, it being also possible for R₃ to be substituted with one or more C₁-C₁₄ alkyl radicals;
R₄ denotes a hydrogen atom, a methyl or ethyl radical, an optionally hydroxylated, linear or branched, saturated or unsaturated C₃-C₅₀ hydrocarbon radical or a radical -CH₂-CHOH-CH₂-O-R₆ in which R₆ denotes a C₁₀-C₂₆ hydrocarbon radical or a radical R₈-O-CO-(CH₂)ₚ, R₈ denotes a C₁-C₂₀ hydrocarbon radical, p being an integer varying from 1 to 12,
R₅ denotes a hydrogen atom or an optionally mono- or polyhydroxylated, linear or branched, saturated or unsaturated C₁-C₃₀ hydrocarbon radical, it being possible for the hydroxyl(s) to be etherified with a (glycosyl)ₙ, (galactosyl)ₘ, sulphogalactosyl, phosphorylethylamine or phosphorylethylammonium radical,
with the proviso that when R₃ and R₅ denote hydrogen or when R₃ denotes hydrogen and R₅ denotes methyl, then R₄ does not denote a hydrogen atom, or a methyl or ethyl radical.

4. Anhydrous paste according to any one of the preceding claims, **characterized in that** the content of ceramide-type compound represents from 0.001 to 5% by weight, more particularly from 0.01 to 3% by weight, and preferably from 0.05 to 1% by weight relative to the weight of the anhydrous paste.

5. Anhydrous paste according to any one of the preceding claims, **characterized in that** the organic inert liquid is chosen from polydecenes of formula C₁₀ₙH_{[(20n)+2]} in which n varies from 3 to 9 and preferably from 3 to 7, fatty acid or fatty alcohol esters, C₁₂-C₂₄ fatty acid esters or diesters of sugars, cyclic ethers or cyclic esters, silicone oils, mineral oils or vegetable oils.

6. Paste according to the preceding claim, **characterized in that** the organic inert liquid is chosen from the group consisting of the polydecenes of formula C₁₀ₙH_{[(20n)+2]} in which n varies from 3 to 9 and preferably from 3 to 7, and fatty acid or fatty alcohol esters.

7. Anhydrous paste according to any one of the preceding claims, **characterized in that** the content of organic inert liquid varies from 5 to 60% by weight, preferably from 10 to 50% by weight relative to the weight of the anhydrous paste.

8. Anhydrous paste according to any one of the preceding claims, **characterized in that** the peroxygenated salt is chosen from persulphates, perborates and percarbonates of ammonium or alkali metals, such as potassium, sodium; magnesium peroxide, alone or as a mixture.

9. Anhydrous paste according to the preceding claim, **characterized in that** the peroxygenated salt content varies from 10 to 70% by weight, preferably from 20 to 60% by weight relative to the weight of the anhydrous paste.

10. Anhydrous paste according to any one of the preceding claims, **characterized in that** the alkaline agent is chosen from urea, silicates, phosphates and bicarbonates of alkali or alkaline-earth metals, and ammonia precursors.

11. Anhydrous paste according to the preceding claim, **characterized in that** the content of alkaline agent varies from 0.01 to 40% by weight, preferably from 0.1 to 30% by weight relative to the weight of the anhydrous paste.

12. Method for bleaching human keratinous fibres, and in particular the hair, comprising the steps consisting:
- in mixing, immediately before use, an anhydrous paste defined according to any one of Claims 1 to 11, with an aqueous hydrogen peroxide composition,
- in applying the mixture obtained to the area of the fibres to be bleached,
- in leaving in for a sufficient period to obtain the desired bleaching,
- in removing the bleaching mixture by rinsing with water followed by washing with a shampoo, and then optionally drying.

13. Multicompartment device for bleaching human keratinous fibres, and more particularly the hair, **characterized in that** it comprises at least two compartments of which one comprises an anhydrous paste according to any one of Claims 1 to 11, and the other an aqueous hydrogen peroxide composition.

## Patentansprüche

1. Wasserfreie Paste zum Entfärben von menschlichen Keratinfasern und insbesondere Haaren, die mindestens eine inerte organische Flüssigkeit, mindestens ein Peroxysalz und mindestens ein Alkalisierungsmittel enthält, **dadurch gekennzeichnet, dass** sie mindestens eine Verbindung vom Ceramidtyp enthält.

2. Wasserfreie Paste nach dem vorhergehenden Anspruch, **dadurch gekennzeichnet, dass** die Verbindung vom Ceramidtyp unter den Ceramiden und/oder Glycoceramiden und/oder Pseudoceramiden ausgewählt ist.

3. Wasserfreie Paste nach einem der vorhergehenden Ansprüche, **dadurch gekennzeichnet, dass** die Verbindung vom Ceramidtyp unter den Verbindungen der folgenden Formel (A) ausgewählt ist: 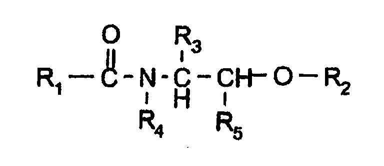 worin bedeuten:
R₁ bedeutet:
- entweder eine geradkettige oder verzweigte, gesättigte oder ungesättigte Kohlenwasserstoffgruppe mit 1 bis 50 und vorzugsweise 5 bis 50 Kohlenstoffatomen, wobei diese Gruppe mit einer oder mehreren Hydroxygruppen substituiert sein kann, die gegebenenfalls mit einer Säure R₇COOH verestert sind, wobei R₇ eine gesättigte oder ungesättigte, geradkettige oder verzweigte, gegebenenfalls ein- oder mehrfach hydroxylierte Kohlenwasserstoffgruppe mit 1 bis 35 Kohlenstoffatomen bedeutet, wobei die Hydroxygruppe(n) der Gruppe R₇ mit einer gesättigten oder ungesättigten, geradkettigen oder verzweigten, gegebenenfalls ein- oder mehrfach hydroxylierten Fettsäure mit 1 bis 35 Kohlenstoffatomen verestert sein können,
- oder eine Gruppe R"-(NR-CO)-R', wobei R ein Wasserstoffatom oder eine ein- oder mehrfach hydroxylierte und vorzugsweise einfach hydroxylierte Kohlenwasserstoffgruppe mit 1 bis 20 Kohlenstoffatomen bedeutet, wobei die Gruppen R' und R" Kohlenwasserstoffgruppen sind, deren Summe der Kohlenstoffatome im Bereich von 9 bis 30 liegt, wobei R' eine zweiwertige Gruppe ist,
- oder eine Gruppe R₈-O-CO-(CH₂)p, wobei R₈ eine Kohlenwasserstoffgruppe mit 1 bis 20 Kohlenstoffatomen ist und p eine ganze Zahl von 1 bis 12 bedeutet;
R₂ ist unter einem Wasserstoffatom, einer Gruppe vom Saccharidtyp und insbesondere einer Gruppe (Glycosyl)ₙ, (Galactosyl)ₘ oder Sulfogalactosyl, einem Sulfatrest oder Phosphatrest, einer Phosphorylethylamingruppe und einer Phosphorylethylammoniumgruppe ausgewählt, worin n eine ganze Zahl von 1 bis 4 und m eine ganze Zahl von 1 bis 8 bedeutet;
R₃ ist ein Wasserstoffatom oder eine gesättigte oder ungesättigte, hydroxylierte oder nicht hydroxylierte Kohlenwasserstoffgruppe mit 1 bis 33 Kohlenstoffatomen, wobei die Hydroxygruppe(n) mit einer anorganischen Säure oder einer Säure R₇COOH verestert sein können, wobei R₇ die oben angegebenen Bedeutungen aufweist, wobei die Hydroxygruppe(n) mit einer Gruppe (Glycosyl)ₙ, (Galactosyl)ₘ, Sulfogalactosyl, Phosphorylethylamin oder Phosphorylethylammonium verethert sein können und wobei R₃ auch mit einer oder mehreren C₁₋₄-Alkylgruppen substituiert sein kann;
R₄ bedeutet ein Wasserstoffatom, Methyl, Ethyl, eine gesättigte oder ungesättigte, geradkettige oder verzweigte, gegebenenfalls hydroxylierte Kohlenwasserstoffgruppe mit 3 bis 50 Kohlenstoffatomen oder eine Gruppe -CH₂-CHOH-CH₂-O-R₆, wobei R₆ eine Kohlenwasserstoffgruppe mit 10 bis 26 Kohlenstoffatomen oder eine Gruppe R₈-O-CO-(CH)ₚ bedeutet, worin R₈ eine C₁₋₂₀-Kohlenwasserstoffgruppe ist und p eine ganze Zahl von 1 bis 12 bedeutet;
R₅ bedeutet ein Wasserstoffatom oder eine gesättigte oder ungesättigte, geradkettige oder verzweigte, gegebenenfalls ein- oder mehrfach hydroxylierte Kohlenwasserstoffgruppe mit 1 bis 30 Kohlenstoffatomen, wobei die Hydroxygruppe(n) mit einer Gruppe (Glycosyl)ₙ, (Galactosyl)ₘ, Sulfogalactosyl, Phosphorylethylamin oder Phosphorylethylammonium verethert sein können, mit der Maßgabe, dass R₄ kein Wasserstoffatom, keine Methyl- oder Ethylgruppe bedeutet, wenn R₃ und R₅ Wasserstoff bedeuten oder wenn R₃ Wasserstoff und R₅ Methyl bedeutet.

4. Wasserfreie Paste nach einem der vorhergehenden Ansprüche, **dadurch gekennzeichnet, dass** der Mengenanteil der Verbindung vom Ceramidtyp im Bereich von 0,001 bis 5 Gew.-%, insbesondere 0,01 bis 3 Gew.-% und vorzugsweise 0,05 bis 1 Gew.-%, bezogen auf die wasserfreie Paste, liegt.

5. Wasserfreie Paste nach einem der vorhergehenden Ansprüche, **dadurch gekennzeichnet, dass** die organische inerte Flüssigkeit unter den Polydecenen der Formel C₁₀ₙH_{[(20n)+2]}, worin n im Bereich von 3 bis 9 und vorzugsweise 3 bis 7 liegt, Estern von Fettalkoholen oder Fettsäuren, Estern oder Diestern von Zuckern und C₁₂₋₂₄-Fettsäuren, cyclischen Ethern oder cyclischen Estern, Siliconölen, Mineralölen oder pflanzlichen Ölen ausgewählt ist.

6. Paste nach dem vorhergehenden Anspruch, **dadurch gekennzeichnet, dass** die inerte organische Flüssigkeit unter den Polydecenen der Formel C₁₀ₙH_{[(20n)+2]}, worin n im Bereich von 3 bis 9 und vorzugsweise 3 bis 7 liegt, und den Estern von Fettalkoholen oder Fettsäuren ausgewählt ist.

7. Wasserfreie Paste nach einem der vorhergehenden Ansprüche, **dadurch gekennzeichnet, dass** der Mengenanteil der organischen inerten Flüssigkeit im Bereich von 5 bis 60 Gew.-% und vorzugsweise 10 bis 50 Gew.-%, bezogen auf das Gesamtgewicht der wasserfreien Paste, liegt.

8. Wasserfreie Paste nach einem der vorhergehenden Ansprüche, **dadurch gekennzeichnet, dass** das Peroxysalz einzeln oder in Form von Gemischen unter den Persulfaten, Perboraten und Percarbonaten von Ammonium oder Alkalimetallen, wie Kalium, Natrium; Magnesiumperoxid ausgewählt ist.

9. Wasserfreie Paste nach dem vorhergehenden Anspruch, **dadurch gekennzeichnet, dass** der Mengenanteil des Peroxysalzes im Bereich von 10 bis 70 Gew.-% und vorzugsweise 20 bis 60 Gew.-%, bezogen auf die wasserfreie Paste, liegt.

10. Wasserfreie Paste nach einem der vorhergehenden Ansprüche, **dadurch gekennzeichnet, dass** das Alkalisierungsmittel unter Harnstoff, Silicaten, Phosphaten, Bicarbonaten von Alkalimetallen oder Erdalkalimetallen und Ammoniakvorläufern ausgewählt ist.

11. Wasserfreie Paste nach dem vorhergehenden Anspruch, **dadurch gekennzeichnet, dass** der Mengenanteil des Alkalisierungsmittels im Bereich von 0,01 bis 40 Gew.-% und vorzugsweise 0,1 bis 30 Gew.-%, bezogen auf das Gewicht der wasserfreien Paste liegt.

12. Verfahren zum Entfärben von menschlichen Keratinfasern und insbesondere Haaren, das die folgenden Schritte umfasst:
- Vermischen einer wasserfreien Paste nach einem der Ansprüche 1 bis 11 unmittelbar vor der Anwendung mit einer wässrigen Wasserstoffperoxidzusammensetzung,
- Aufbringen des erhaltenen Gemisches auf den Bereich der Fasern, der entfärbt werden soll,
- Einwirkenlassen während einer Zeitspanne, die ausreichend ist, um die gewünschte Entfärbung zu erhalten,
- Entfernen des Gemisches zum Entfärben durch Spülen mit Wasser und anschließend Waschen mit Haarwaschmittel, worauf gegebenenfalls getrocknet wird.

13. Vorrichtung mit mehreren Abteilungen zum Entfärben von menschlichen Keratinfasern und insbesondere Haaren, **dadurch gekennzeichnet, dass** sie mindestens zwei Abteilungen aufweist, wobei eine Abteilung eine wasserfreie Paste nach einem der Ansprüche 1 bis 11 enthält und die andere eine wässrige Wasserstoffperoxidzusammensetzung.
